(19) Europäisches Patentamt
European Patent Office
Office européen des brevets

(11) Publication number: **0 014 792**
**B1**

(12) # EUROPEAN PATENT SPECIFICATION

(45) Date of publication of patent specification: **01.12.82**

(51) Int. Cl.³: **C 07 D 209/44,**
**A 61 K 31/40**

(21) Application number: **79301248.5**

(22) Date of filing: **28.06.79**

(54) **Tetrahydro-2H-benzo(c)pyrroles, their preparation, pharmaceutical compositions containing them and the compounds for use as therapeuticals.**

(30) Priority: **22.01.79 US 5063**
**15.03.79 US 20560**

(43) Date of publication of application:
**03.09.80 Bulletin 80/18**

(45) Publication of the grant of the patent:
**01.12.82 Bulletin 82/48**

(84) Designated Contracting States:
**DE GB IT NL SE**

(56) References cited:
**GB - A - 984 517**

(73) Proprietor: **ELI LILLY AND COMPANY**
**307, East McCarty Street**
**Indianapolis Indiana 46285 (US)**

(72) Inventor: **Bach, Nicholas James**
**4269, Burkhard Street, E Drive**
**Indianapolis Indiana (US)**
Inventor: **Kornfeld, Edmund Carl**
**5159 East 76th Court**
**Indianapolis Indiana (US)**

(74) Representative: **Crowther, Terence Roger**
**European Patent Attorney et al,**
**Erl Wood Manor**
**Windlesham Surrey GU20 6PH (GB)**

EP 0 014 792 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Tetrahydro-2H-benzo(c)pyrroles, their preparation, pharmaceutical compositions containing them and the compounds for use as therapeuticals

This invention provides substituted 4,5,6,7-tetrahydro-2H-benzo[c]pyrroles of the general formula

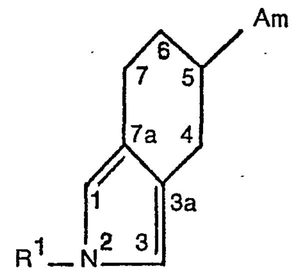

I

wherein

$R^1$ is H or $R^2$;

Am is $N(R^2)_2$; and

each $R^2$ is independently allyl, methyl, ethyl, or n-propyl; and

the pharmaceutically-acceptable acid addition salts thereof.

The compounds of formula I are prepared by reacting a 5-amino-4,5,6,7-tetrahydro-2H-benzo[c]pyrrole compound of the formula

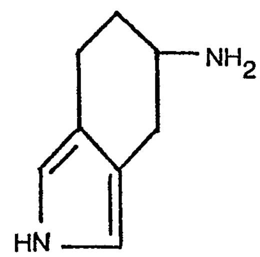

V

with an appropriate aldehyde and then reducing with a metal hydride; or with an allyl or appropriate alkyl halide, then an appropriate acyl halide followed by reducing with a metal hydride; to provide the compounds of formula I wherein $R^1$ is H, and Am is $N(R^2)_2$;

followed by reacting with an allyl or appropriate alkyl halide and base to provide the compounds of formula I wherein $R^1$ is $R^2$, and Am is $N(R^2)_2$.

The compounds of formula I are useful as dopamine agonists including their use in treating Parkinson's Syndrome and as prolactin inhibitors.

Additionally, the following compounds are useful as novel intermediates and have the general formula

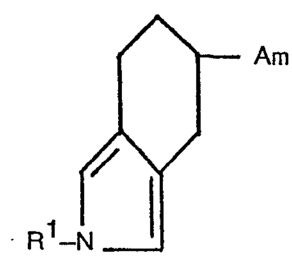

IA

wherein

$R^1$ is H or

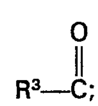

Am is $NH_2$ or

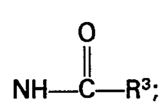

$R^3$ is methyl, ethyl or n-propyl; except that $R^1$ is not

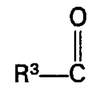

when Am is $NH_2$; and the acid addition salts thereof when Am is $NH_2$.

The pharmaceutically-acceptable acid addition salts of the compounds of formulae I and IA include salts derived from non-toxic inorganic acids such as: hydrochloric acid, nitric acid, phosphoric acid, sulfuric acid, hydrobromic acid, hydriodic acid, nitrous acid and phosphorous acid, as well as salts derived from non-toxic organic acids such as aliphatic mono and dicarboxylic acids, phenyl-substituted alkanoic acids, hydroxy alkanoic and alkandioic acids, aromatic acids and aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycollate, malate, tartrate, methanesulfonate, propanesulfonate, naphthalene-1-sulfonate and naphthalene-2-sulfonate salts.

Acid addition salts of those intermediate compounds of formula IA are not restricted to those formed with non-toxic anions since the chief use of such salts is for isolation and purification of the intermediates involved.

Illustrative compounds coming within the scope of formula I above include:
dl-5-dimethylamino-4,5,6,7-tetrahydro-2H-benzo[c]pyrrole methane sulfonate.
dl-2-methyl-5-diethylamino-4,5,6,7-tetrahydro-2H-benzo[c]pyrrole maleate.
dl-5-diallylamino-4,5,6,7-tetrahydro-2H-benzo[c]pyrrole sulfate.
dl-2-ethyl-5-di(n-propyl)amino-4,5,6,7-tetrahydro-2H-benzo[c]pyrrole hydrochloride.
N,N,2-trimethyl-dl-5-amino-4,5,6,7-tetrahydro-2H-benzo[c]pyrrole.
N-methyl-N-allyl-dl-5-amino-4,5,6,7-tetrahydro-2H-benzo[c]pyrrole.
dl-2-allyl-5-dimethylamino-4,5,6,7-tetrahydro-2H-benzo[c]pyrrole.
N-methyl-N-ethyl-dl-5-amino-4,5,6,7-tetrahydro-2H-benzo[c]pyrrole.
N-methyl-N-n-propyl-dl-2-methyl-5-amino-4,5,6,7-tetrahydro-2H-benzo[c]pyrrole.
N-allyl-N-n-propyl-dl-5-amino-4,5,6,7-tetrahydro-2H-benzo[c]pyrrole.

While the compounds represented by formula I have been named as 4,5,6,7-tetrahydro-2H-benzo[c]pyrroles, an alternative name can be used; i.e., the compounds can be named as 4,5,6,7-tetrahydroisoindoles.

The presence of a substituent at C—5 in the benzo[c]pyrrole or isoindole ring introduces a center of assymetry into those molecules. Thus, compounds represented by formula I include two optical isomers occurring as a dl pair or racemate. Resolution of a dl pair of formula I into its optical antipodes can be accomplished by procedures known to those skilled in the art.

A process for preparing the novel intermediate compounds of the general formula

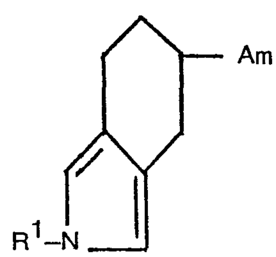

IA

wherein

$R^1$ is H or

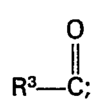

Am is $NH_2$ or

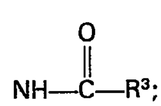

$R^3$ is methyl, ethyl, or n-propyl; except that $R^1$ is not

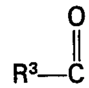

when Am is $NH_2$; and the acid addition salts thereof when Am is $NH_2$, which comprises reacting either

(A)

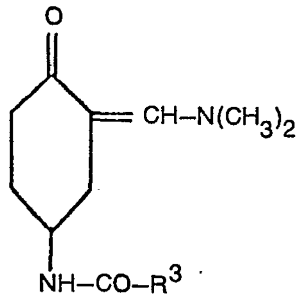

III

wherein

$R^3$ is defined as above, with sodium glycinate followed by a ring closure reaction in the presence of, for example, acetic anhydride, to provide the compounds of formula IA where Am is

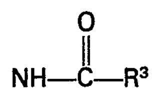

and $R^1$ is

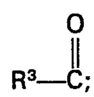

followed by reacting the product with base to provide the compounds of formula IA where Am is $NH_2$ and $R^1$ is H; or

(B)

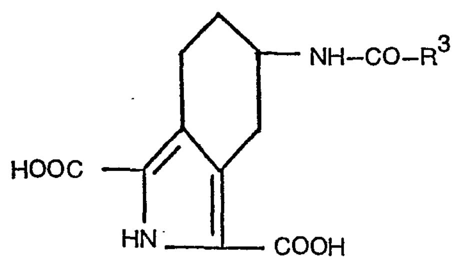

XI

wherein

$R^3$ is defined as above, with copper powder in the presence of quinoline to provide the compounds of formula IA where Am is

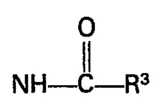

and $R^1$ is H; followed by basic hydrolysis to provide the compounds of formula IA where Am is $NH_2$ and $R^1$ is H.

The compounds of formula I containing an amino group (Am) at C—5 in the tetrahydro-2H-benzo[c]pyrrole ring system can be prepared by at least two different procedures starting from a 4-acylaminocyclohexanone, formula II. The first of these is illustrated in Reaction Sequence I below:

REACTION SEQUENCE I

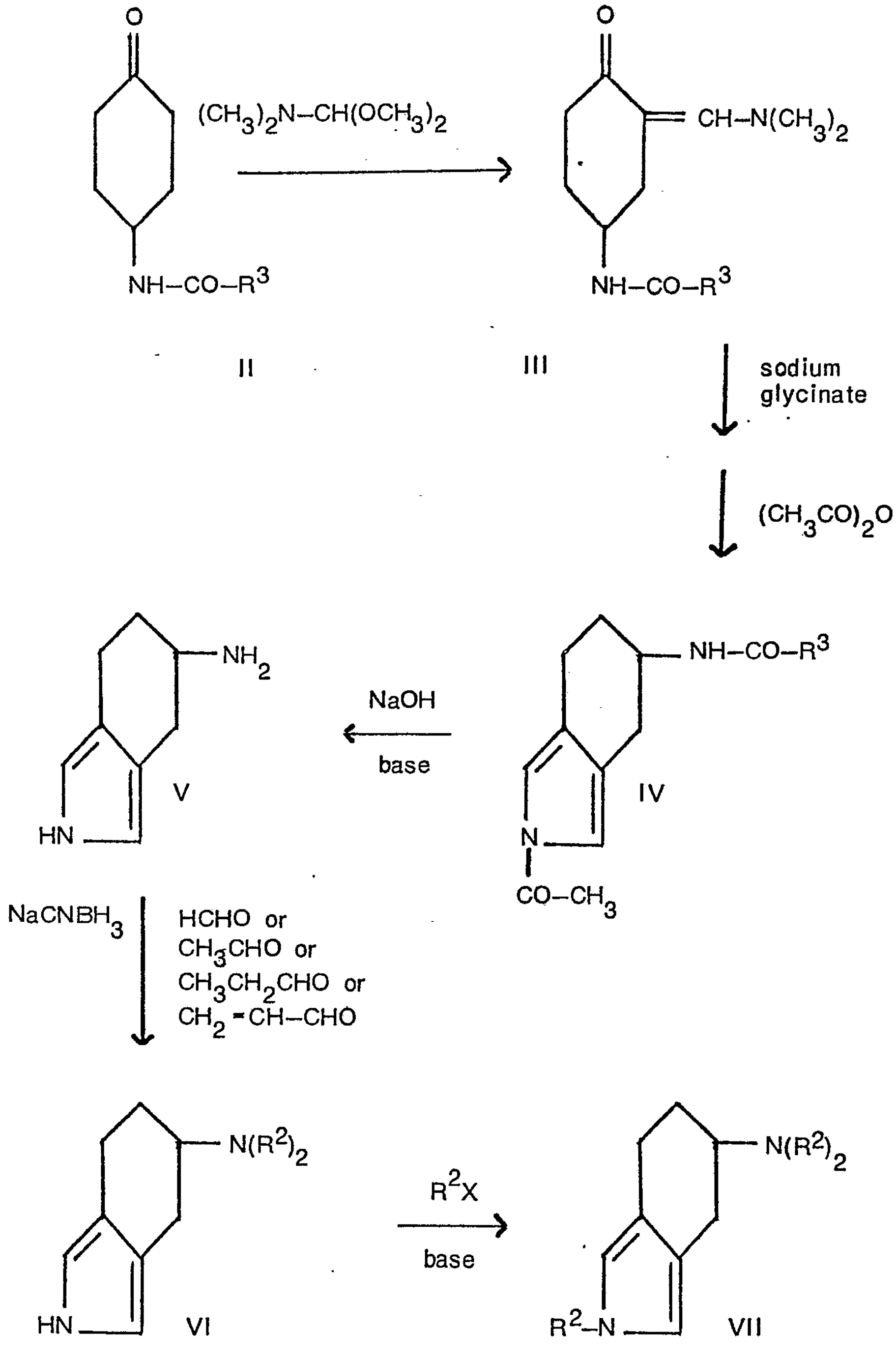


II III IV V VI VII

According to Reaction Scheme I, a 4-acylaminocyclohexanone, prepared by the procedure of Fraser and Swingle, *Can. J. Chem., 48,* 2065 (1970) is reacted with the dimethylacetal of dimethylformamide to yield a 2-dimethylaminomethylene-4-acylaminocyclohexanone (III). Reacting this compound with sodium glycinate followed by a ring closure reaction in the presence of, for example, acetic anhydride yields, when $R^3$ is methyl, dl-2-acetyl-5-acetamido-4,5,6,7-tetrahydro-2H-benzo[c]-pyrrole (IV). Treatment of this latter compound with base gives dl-5-amino-4,5,6,7-tetrahydro-2H-benzo[c]pyrrole (V). This latter compound can be preferentially substituted on the amino group at C—5 using a reductive procedure; i.e., reaction with an appropriate aldehyde (formaldehyde, acetaldehyde, acrolein, or propionaldehyde) in the presence of a metal hydride reducing agent, such as sodium cyanoborohydride. The dialkylated compound, for instance, dl-5-di(n-propyl)amino-4,5,6,7-tetrahydro-2H-benzo[c]pyrrole (VI) can also be substituted on the pyrrole ring nitrogen under basic conditions using an allyl or appropriate alkyl halide $R^2X$ (methyl iodide, allyl chloride, ethyl bromide, or the like) to yield a dl-2-($C_1$—$C_3$ alkyl or allyl)-5-disubstituted-amino-4,5,6,7-tetrahydro-2H-benzo[c]pyrrole of formula VII.

Other methods of substitution of the amine groups at C—5 can be employed in addition to the reductive procedure illustrated above for converting V to VI. For example, direct reaction with an allyl or appropriate alkyl halide, particularly an iodide, followed by a reaction of the thus-formed secondary amine with a suitable acyl halide; i.e., acetyl chloride or crotyl chloride followed by reduction again with a metal hydride reducing agent, such as lithium aluminum hydride or diborane, yields a 5-disubstituted amino compound. This latter procedure particularly lends itself to the preparation of unsymmetrically substituted amine groups at C—5.

A second synthetic procedure is available for preparing the compounds of formula I as illustrated in Reaction Sequence II below:

REACTION SEQUENCE II

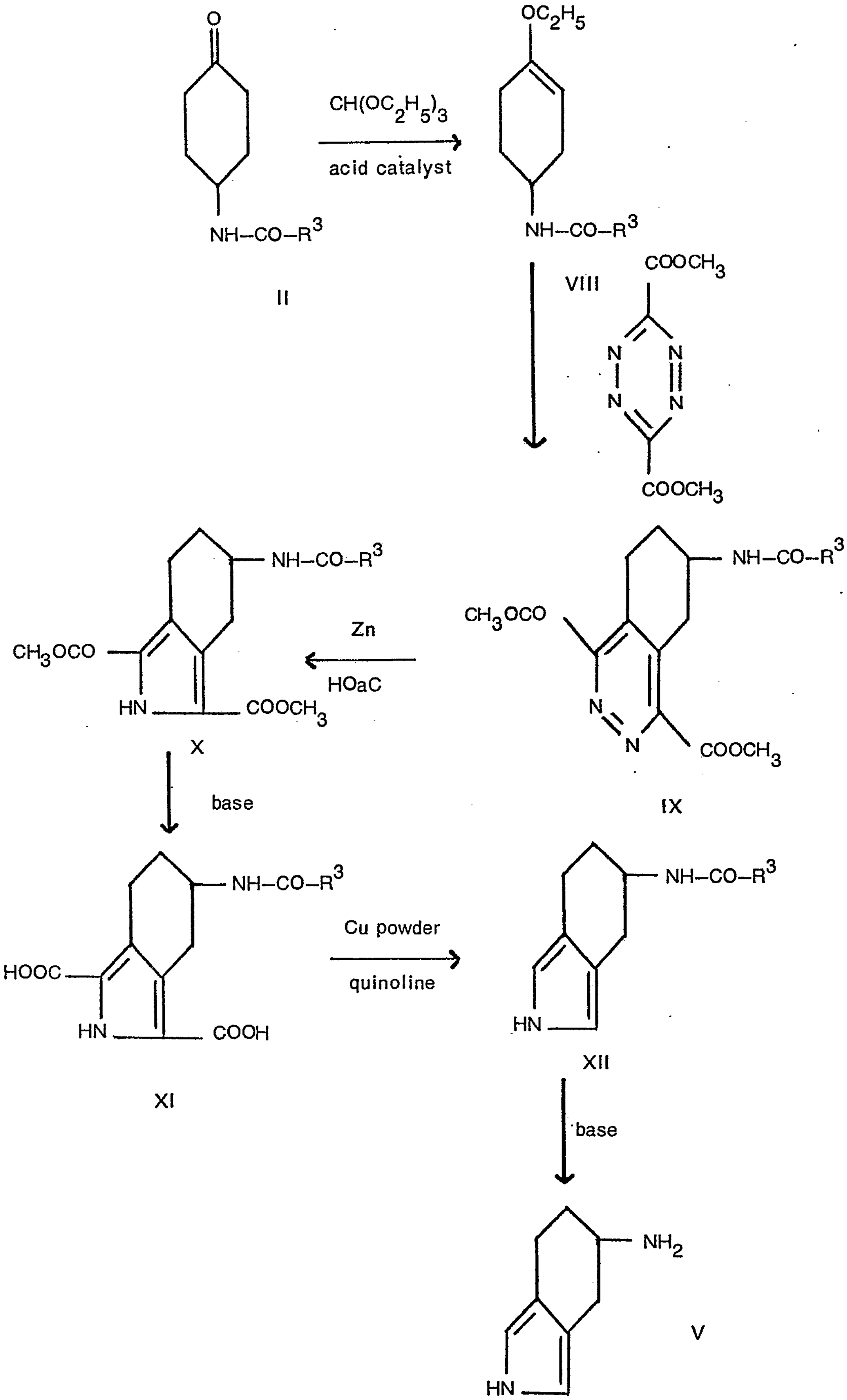


II VIII IX X XI XII V

According to Reaction Sequence II, the same starting material 4-acylaminocyclohexanone, is employed as in Reaction Sequence I. The reaction of this starting material with ethyl orthoformate in the presence of an acid catalyst such as p-toluene sulfonic acid produces an enol ether; i.e., a 4-acylamino-1-ethoxycyclohexene (VII). Reaction of this derivative with 1,2,4,5-tetrazine dicarboxylic acid, dimethyl ester [prepared by the procedure of Sauer, et al. *Chem. Ber., 98,* 1435 (1965)] yields a pyridazine diester (IX). Reduction of this diester with zinc in acetic acid or other suitable metal-acid reducing system causes a ring contraction to yield dl-1,3-dicarbomethoxy-5-acylamino-4,5,6,7-tetrahydro-2H-benzo[c]pyrrole (X). Selective hydrolysis of the diester with base yields the corresponding dicarboxylic acid (XI) which, on decarboxylation with copper powder in the presence of quinoline, gives dl-5-acylamino-4,5,6,7-tetrahydro-2H-benzo[c]pyrrole (XIII). Hydrolysis under basic conditions of the acyl group then yields the free amine, dl-5-amino-4,5,6,7-tetrahydro-2H-benzo[c]pyrrole (identical to compound V from Reaction Sequence I). The acyl protecting groups which can be used in the 4-acylamino cyclohexanone starting material are acetyl, propionyl and butyryl.

This invention is further illustrated by the following preparative examples:

Example A

PREPARATION OF DL-5-AMINO-4,5,6,7-TETRAHYDRO-2H-BENZO[c]PYRROLE

A reaction mixture was prepared from 15.5 g. of 4-acetamidocyclohexanone [prepared by the procedure of Fraser and Swingle, *Can. J. Chem., 48,* 2065 (1970)], 80 g. of the dimethylacetal of dimethylformamide, 1.5 ml. of triethylamine and 500 ml. of benzene. The benzene was distilled therefrom over a 1.5 hour period until the volume was reduced to about 1/2 of the original volume. An additional 250 ml. of benzene were added. The reaction mixture was heated just below the boiling point of benzene for about 2 hours and was then distilled again until the volume was about one-half of that originally present (250 ml). The above process was repeated once more except that the volume was reduced to one-third of the original volume (167 ml.). The reaction mixture was then cooled and filtered. The filter cake consisted of dl-4-acetamido-2-dimethylaminomethylenecyclohexanone formed in the above reaction; weight = 6.45 g. Evaporation of the filtrate to dryness yielded a residue, chromatography of a chloroform solution of which over 200 g. of Florisil using chloroform containing increasing amounts of methanol (0—5%) as an eluant, yielded more dl-4-acetamido-2-dimethylaminomethylenecyclohexanone; M.P. = 132—133°C. (from benzene); yield = 5.55 g.; total yield = 12 g.

| | | | | | | | |
|---|---|---|---|---|---|---|---|
| Analysis Calc. | : | C, | 62.83; | H, | 8.63; | N, | 13.32 |
| Found | : | C, | 63.07; | H, | 8.38; | N, | 13.12 |

Potassium glycinate was prepared by reacting 9 g. of glycine and 6.7 g. of potassium hydroxide in 400 ml. of anhydrous ethanol. 22.6 g. of dl-4-acetamido-2-dimethylaminomethylenecyclohexanone were added thereto and the resulting mixture heated to refluxing temperature under a nitrogen atmosphere for 1.75 hours. The reaction mixture was cooled, diluted with ether, and filtered. The filter cake, which weighed 28.7 g., was added to 400 ml. of acetic anhydride and the resulting mixture heated to reflux temperature under a nitrogen atmosphere for one hour. The reaction mixture was cooled and the volatile constituents removed by evaporation in vacuo. The resulting residue was suspended in chloroform and filtered. The filtrate was chromatographed over 350 g. of Florisil (Registered Trade Mark), using chloroform containing increasing amounts (0—2%) of methanol as the eluant. Fractions shown by TLC to contain dl - 2 - acetyl - 5 - acetamido - 4,5,6,7 - tetrahydro - 2H - benzo[c]pyrrole formed in above reaction were combined and the solvent evaporated therefrom. Crystallization of the residue from ether yielded purified dl-2-acetyl-5-acetamido-4,5,6,7-tetrahydro-2H-benzo[c]pyrrole melting at 151—153°C.; yield = 17.7 g.

| | | | | | | | |
|---|---|---|---|---|---|---|---|
| Analysis Calc. : | C, | 65.43; | H, | 7.32; | N, | 12.72; |
| Found : | C, | 65.72; | H, | 7.34; | N, | 12.45. |

A hydrolysis mixture was prepared from 5.1 g. of dl-2-acetyl-5-acetamido-4,5,6,7-tetrahydro-2H-benzo[c]pyrrole, 50 g. of sodium hydroxide, 50 ml. of water, and 200 ml. of ethanol. The mixture was heated to refluxing temperature under a nitrogen atmosphere for about 16 hours and was then cooled. The cooled mixture was diluted with water. The alkaline aqueous mixture was extracted several times with methylene dichloride, the methylene dichloride extracts were combined and the combined extracts washed with saturated aqueous sodium chloride and dried. Evaporation of the solvent therefrom yielded a residue comprising dl-5-amino-4,5,6,7-tetrahydro-2H-benzo[c]pyrrol formed in the above reaction. A chloroform solution of the residue was filtered through 105 g. of alumina (grade II). Concentration of the resulting filtrate yielded 2.52 g. of a yellow solid comprising dl-5-amino-4,5,6,7-tetrahydro-2H-benzo[c]pyrrole.

## Example B

PREPARATION OF dl-5-ACETAMIDO-4,5,6,7-TETRAHYDRO-2H-BENZO[c]PYRROLE

To a solution of 6.7 g. of 4-acetamidocyclohexanone [prepared by the method of Fraser and Swingle, *Can. J. Chem., 48,* 2065 (1970)], in 150 ml. of anhydrous ethanol were added 25 ml. of ethyl orthoformate containing a few crystals of p-toluenesulfonic acid monohydrate. The reaction mixture was stirred at ambient temperature for about 16 hours, after which time the volatile constituents were removed by evaporation in vacuo. The residue comprising the diethylketal was dissolved in 200 ml. of toluene and the toluene was removed by distillation under a nitrogen atmosphere, until all of the diethylketal had been converted to the 1-ethoxycyclohexene derivative. The solution was cooled, washed with aqueous sodium bicarbonate and dried. Evaporation of the toluene yielded a residue comprising 4-acetamido-1-ethoxycyclohexene melting at 100—102°C. after recrystallization from an ether-hexane solvent mixture; yield = 6.2 g.

A solution of 3 g. of 4-acetamido-1-ethoxycyclohexene in 40 ml. of dioxane was added slowly to a solution of 3.2 g. of 1,2,4,5-tetrazine dicarboxylic ester [prepared by the method of Sauer, et al., *Chem. Ber., 98,* 1435 (1965)] in 100 ml. of dioxane. The reaction mixture was stirred at ambient temperature for about 3 days after which time TLC indicated one major spot with several minor spots present. The reaction mixture was evaporated in vacuo, the resulting residue dissolved in chloroform, and the chloroform solution chromatographed over 200 g. of Florisil using chloroform containing increasing amounts (2—5%) of methanol as the eluant. Fractions shown to contain a single major spot material by TLC were combined and the solvent removed from the combined fractions in vacuo. The residue was crystallized by triturating with ether; mp = 137—139°C; yield = 3.21 g. Recrystallization of the residue from the ether-methanol solvent mixture yielded purified dl-6-acetamido-1,4-di(carbomethoxy)-5,6,7,8-tetrahydrobenzo[d]pyridazine melting at 143—144°C.

| | | | | | | |
|---|---|---|---|---|---|---|
| Analysis Calc. : | C, | 54.72; | H, | 5.58; | N, | 13.67; |
| Found : | C, | 54.75; | H, | 5.64; | N, | 13.49. |

A solution was prepared by adding 2.59 g. of dl-6-acetamido-1,4-di(carbomethoxy)-5,6,7,8-tetrahydrobenzo[d]pyridazine to 100 ml. of glacial acetic acid. 5 g. of zinc dust were added and the resulting mixture stirred at ambient temperature for about 1 day. An additional 5 g. of zinc dust were added after 6 hours. The reaction mixture was then filtered to remove unreacted zinc dust and the resulting filtrate poured over ice. The filtrate was made basic with 14N aqueous ammonium hydroxide and the alkaline mixture extracted several times with a chloroform-isopropanol solvent combination. The organic extracts were separated and combined, and the combined extracts washed with saturated aqueous sodium chloride and then dried. Evaporation of the solvent therefrom yielded dl-5-acetamido-1,3-di(carbomethoxy)-4,5,6,7-tetrahydro-2H-benzo[c]pyrrole formed in the above reaction; yield = 1.83 g. Recrystallization from methanol yielded crystalline material melting at 231—232°C.

| | | | | | | |
|---|---|---|---|---|---|---|
| Analysis Calc. : | C, | 57.14; | H, | 6.16; | N, | 9.52 |
| Found : | C, | 57.05; | H, | 5.99; | N, | 9.26 |

A reaction mixture was prepared containing 1.8 g. of dl-5-acetamido-1,3-di(carbomethoxy)-4,5,6,7-tetrahydro-2H-benzo[c]pyrrole, 80 ml. of THF and 20 ml. of 2N aqueous sodium hydroxide. The reaction mixture was heated to refluxing temperature under a nitrogen atmosphere for about 3 hours after which time it was cooled and the volatile constituents evaporated therefrom in vacuo. The residue was dissolved in 25 ml. of water and the aqueous solution made acidic by the addition of 1N aqueous hydrochloric acid. The diacid, dl-5-acetamido-1,3-dicarboxy-4,5,6,7-tetrahydro-2H-benzo[c]pyrrole, formed in the above hydrolysis being insoluble in the acidic layer precipitated and was collected by filtration. Recrystallization from a benzene-methanol solvent mixture yielded crystalline diacid melting at 233—235°C. with decomposition.

| | | | | | | | |
|---|---|---|---|---|---|---|---|
| Analysis Calc. | : | C, | 54.13; | H, | 5.30; | N, | 10.52 |
| Found | : | C, | 53.90; | H, | 5.37; | N, | 10.45 |

A reaction mixture, prepared from 850 mg. of dl-5-acetamido-1,3-dicarboxy-4,5,6,7-tetrahydro-2H-benzo[c]pyrrole, 50 mg. of copper powder and 25 ml. of quinoline, was heated to 200°C. under a nitrogen atmosphere. Gas evolution was noticeable as the temperature approached 150°C. Heating in the range 200—210°C. was carried on for 15 minutes after which time the reaction mixture was poured over ice. The resulting aqueous mixture was extracted with chloroform, and the chloroform extract was separated, washed with 0.1N aqueous hydrochloric acid, 10 percent aqueous sodium hydroxide and finally water. The chloroform was removed therefrom by evaporation in vacuo to yield 0.25 g. of a dark oil as a residue. Chromatography over 15 g. of Florisil using chloroform containing from 0 to 1 percent methanol as the eluant yielded 40 mg. of dl-5-acetamido-4,5,6,7-tetrahydro-2H-benzo[c]pyrrole. The product was identical to the compound prepared by deacetylation of dl-5-acetamido-2-acetyl-4,5,6,7-tetrahydro-2H-benzo[c]pyrrole from Example A.

## FINAL PRODUCTS

### Example 1

### PREPARATION OF dl-5-DIETHYLAMINO-4,5,6,7-TETRAHYDRO-2H-BENZO[c]PYRROLE

A solution was prepared by adding 2.52 g. of dl-5-amino-4,5,6,7-tetrahydro-2H-benzo[c]pyrrole, prepared in Example A, to 100 ml. of methanol. 1.2 g. of sodium cyanoborohydride were added followed by 6 ml. of acetaldehyde. The reaction mixture was stirred at ambient temperature under nitrogen atmosphere for about 16 hours, and was then diluted with aqueous sodium bicarbonate. The aqueous layer was extracted with chloroform, and the chloroform extract separated and washed with saturated aqueous sodium chloride. The chloroform solution was then dried and the solvent removed therefrom by evaporation. The resulting residue was redissolved in chloroform and the chloroform solution chromatographed over 35 g. of Florisil using chloroform containing increasing amounts (2—4%) methanol as the eluant. By combining fractions shown by TLC to contain a material different than starting material, 1.84 g. of solid were obtained which was rechromatographed over Florisil. Combining fractions shown to contain dl-5-diethylamino-4,5,6,7-tetrahydro-2H-benzo[c]pyrrole by TLC followed by evaporation of the solvent from the combined fractions in vacuo yielded a residue weighing 0.66 g. The residue was dissolved in ether and treated with an excess of an ethereal solution of maleic acid. dl-5-diethylamino-4,5,6,7-tetrahydro-2H-benzo[c]pyrrole maleate thus prepared was recrystallized from a methanol-ether solvent mixture to yield purified compound melting at 81—83°C.; yield = 385 mg.

| | | | | | | | |
|---|---|---|---|---|---|---|---|
| Analysis Calc. | : | C, | 62.32; | H, | 7.84; | N, | 9.08 |
| Found | : | C, | 62.37; | H, | 7.57; | N, | 8.94 |

### Example 2

### PREPARATION OF dl-5-DI-n-PROPYLAMINO-4,5,6,7-TETRAHYDRO-2H-BENZO[c]PYRROLE

Following the procedure of Example 1 but substituting propionaldehyde for acetaldehyde, dl-5-di-n-propylamino-4,5,6,7-tetrahydro-2H-benzo[c]pyrrole maleate was prepared, melting at 134—136°C. after recrystallization from an isopropanol-ether solvent mixture.

| | | | | | | | |
|---|---|---|---|---|---|---|---|
| Analysis Calc. | : | C, | 64.26; | H, | 8.39; | N, | 8.33 |
| Found | : | C, | 64.32; | H, | 8.68; | N, | 8.17 |

### Example 3

### PREPARATION OF dl-2-METHYL-5-DI-n-PROPYLAMINO-4,5,6,7-TETRAHYDRO-2H-BENZO[c]PYRROLE

2 millimoles (680 mg) of dl-5-di(n-propyl)amino-4,5,6,7-tetrahydro-2H-benzo[c]pyrrole were dissolved in 75 ml. of dimethylacetamide (DMA). 10 millimoles (1.1 g) potassium tertiary-butoxide were added and the resulting mixture stirred for 20 minutes under nitrogen. Next, a solution of 2.1 millimoles of methyl iodide (0.13 ml) in 5 ml. of DMA was added in dropwise fashion. The resulting reaction mixture was stirred at ambient temperature for 5 hours at which time an additional 0.13 ml of methyl iodide was added and the subsequent mixture stirred for an additional 3.5 hours. The reaction mixture was then diluted with water and the aqueous layer extracted with ethyl acetate. The ethyl acetate layer was separated and washed with water followed by saturated aqueous sodium chloride. The ethyl acetate layer was then dried and the ethyl acetate removed therefrom by evaporation. An ether solution of the resulting residue was chromatographed over 35 g. of Florisil using ether as the eluant. Fractions shown by TLC to contain dl-2-methyl-5-di-n-propylamino-4,5,6,7-tetrahydro-2H-benzo[c]pyrrole were combined and the solvent removed therefrom in vacuo. NMR of the residue thus obtained indicated that the dl-2-methyl-5-di-n-propylamino-4,5,6,7-tetrahydro-2H-benzo[c]pyrrole had been prepared.

As evidence of the utility of the compounds of formula I in the treatment of Parkinson's

Syndrome, it has been found that they affect turning behavior in a test procedure utilizing 6-hydroxydopamine-lesioned rats. In this test, nigro-neostriatal-lesioned rats are employed prepared by the procedure of Ungerstedt and Arbuthnott, *Brain Res, 24,* 485 (1970). A compound having dopamine agonist activity upon injection causes the rats to turn in circles contralateral to the side of the lesion. After a latency period, which varies from compound to compound, the number of turns is counted over a 15-minute period. The compounds are dissolved in water and the aqueous solution injected into the rat by the intraperitoneal route at a dose level of 1 mg/kg. dl-5-Di(n-propyl)amino-4,5,6,7-tetrahydro-2H-benzo[c]pyrrole maleate gave an average of 34 turns per rat with 1/3 of the rats exhibiting turning behavior in the above test.

The compounds of formula I are also useful as prolactin inhibitors and as such can be employed in the treatment of inappropriate lactation, such as postpartum lactation, and of galactorrhea. As evidence of their utility in the treatment of diseases in which it is desirable to reduce the mammalian prolactin level, the compounds of formula I have been shown to inhibit prolactin according to the following procedure.

Adult male rats of the Sprague-Dawley strain weighing about 200 g. were housed in an air-conditioned room with controlled lighting (lights on 6 a.m. — 8 p.m.) and fed lab chow and water *ad libitum.* Each rat received an intraperitoneal injection of 2.0 mg. of reserpine in aqueous suspension 18 hours before administration of the isoindole. The purpose of the reserpine was to keep prolactin levels uniformly elevated. The compounds under test were dissolved in water and were injected intraperitoneally at doses ranging from 5 mg/kg down to 50 mcg/kg. Each compound was administered at each dose level to a group of 10 rats, and a control group of 10 intact males received an equivalent amount of solvent. One hour after treatment all rats were killed by decapitation, and 150 $\mu$l aliquots of serum were assayed for prolactin.

The difference between the prolactin level of the treated rats and prolactin level of the control rats divided by the prolactin level of the control rats gives the percent inhibition of prolactin secretion attributable to the compounds of formula I. These inhibition percentages are given in Table 1 below. In the table, column 1 gives the name of the compound; and columns 2—4 the percent prolactin inhibition at the given dose level.

TABLE 1

| | Percent Inhibition of Prolactin at a Given Dose Level | | |
|---|---|---|---|
| Name of Compound | 5 mg/kg | 500 mcg/kg | 50 mcg/kg |
| dl-5-di(n-propyl)amino-4,5,6,7-tetrahydro-2H-benzo[c]pyrrole maleate | 91 | 47 | 30 |
| dl-5-diethylamino-4,5,6,7-tetrahydro-2H-benzo[c]pyrrole maleate | 23 | 25 | — |

In using the compounds of formula I to inhibit prolactin secretion or to treat Parkinson's syndrome or for other pharmacologic action, a pharmaceutically active compound of formula I above, or a salt thereof with a pharmaceutically-acceptable acid, is administered to a subject suffering from Parkinsonism or in need of having their prolactin level reduced in an amount effective to alleviate some of the symptoms of Parkinsonism or to reduce an elevated prolactin level. Oral administration is preferred. If parenteral administration is used, the injection is preferably by the subcutaneous route using an appropriate pharmaceutical formulation. Other modes of parenteral administration such as intraperitoneal, intramuscular, or intravenous routes are equally effective. In particular, with intravenous or intramuscular administration, a water soluble pharmaceutically-acceptable salt is employed. For oral administration, a pharmaceutically active compound of formula I either as the free base or in the form of a salt thereof can also be mixed with standard pharmaceutical excipients and loaded into empty telescoping gelatin capsules or pressed into tablets. The oral dosage range is from about 0.01 to 10 mg/kg of mammalian weight and the parenteral dosage range from about 0.0025 to 2.5 mg/kg of mammalian weight. Intraperitoneal dosages of 10—30 mg/kg of dl-5-di(n-propyl)amino-4,5,6,7-tetrahydrobenzo[2H]pyrrole maleate in the mouse resulted in no deaths but dosages of 100—300 mg/kg were fatal. Some toxic side effects were observed at the 30 mg/kg dose level.

**Claims**

1. A tetrahydro-2H-benzo[c]pyrrole compound of the general formula

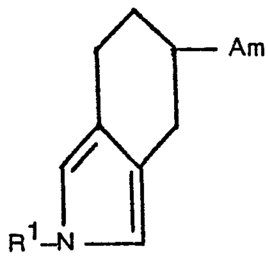

I

wherein

$R^1$ is H or $R^2$;

Am is $N(R^2)_2$; and each $R^2$ is independently allyl, methyl, ethyl, or n-propyl; and the pharmaceutically-acceptable acid addition salts thereof.

2. A compound of claim 1 wherein each $R^2$ is an identical group.

3. dl-5-Diethylamino-4,5,6,7-tetrahydro-2H-benzo[c]pyrrole.

4. dl-5-Di-n-propylamino-4,5,6,7-tetrahydro-2H-benzo[c]pyrrole.

5. dl-2-Methyl-5-di-n-propylamino-4,5,6,7-tetrahydro-2H-benzo[c]pyrrole.

6. A process for preparing a tetrahydro-2H-benzo[c]pyrrole compound of the general formula

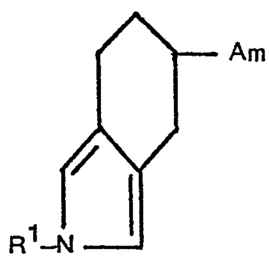

I

wherein

$R^1$ is H or $R^2$;

Am is $N(R^2)_2$; and each $R^2$ is independently allyl, methyl, ethyl, or n-propyl; and the pharmaceutically-acceptable acid addition salts thereof; which comprises reacting a 5-amino-4,5,6,7-tetrahydro-2H-benzo[c]pyrrole compound of the formula

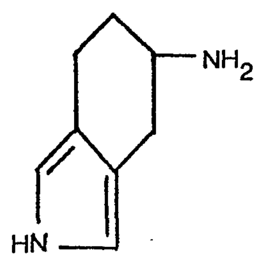

V

with an appropriate aldehyde and then reducing with a metal hydride; or with an allyl or appropriate alkyl halide, then an appropriate acyl halide followed by reducing with a metal hydride; to provide the compounds of formula I wherein $R^1$ is H, and Am is $N(R^2)_2$; followed by reacting with an alkyl halide and base to provide the compounds of formula I wherein $R^1$ is $R^2$, and Am is $N(R^2)_2$.

7. A pharmaceutical composition which comprises a carrier and as active ingredient a compound of formula I as defined in any of claims 1 to 5.

8. A compound of the general formula:

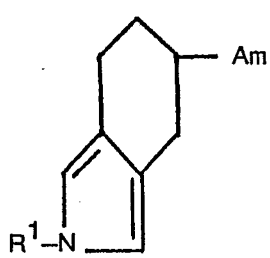

IA

wherein

$R^1$ is H or

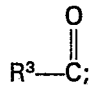

Am is $NH_2$ or

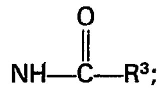

$R^3$ is methyl, ethyl, or n-propyl; except that $R^1$ is not

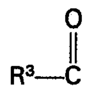

when Am is $NH_2$; and the acid addition salts thereof when Am is $NH_2$.

9. A compound of formula I as defined in claim 1 whenever prepared by the process of claim 6.

10. A compound of Formula I for use in the treatment of Parkinson's Syndrome and as a prolactin inhibitor.

**Revendications**

1. Composé de tétrahydro-2H-benzo[c]pyrrole de formule générale:

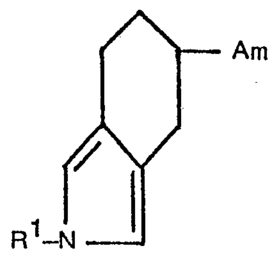

I

dans laquelle

$R^1$ représente H ou $R^2$;

Am représente $N(R^2)_2$; et chaque radical $R^2$ représente indépendamment un groupe allyle, un groupe méthyle, un groupe éthyle ou un groupe n-propyle; de même que les sels d'addition d'acide pharmaceutiquement acceptables de ce composé.

2. Composé suivant la revendication 1, caractérisé en ce que chaque radical $R^2$ est un group identique.

3. dl-5-diéthylamino-4,5,6,7-tétrahydro-2H-benzo[c]pyrrole.

4. dl-5-di-n-propylamino-4,5,6,7-tétrahydro-2H-benzo[c]pyrrole.

5. dl-2-méthyl-5-di-n-propylamino-4,5,6,7-tétrahydro-2H-benzo[c]pyrrole.

6. Procédé de préparation d'un composé de tétrahydro-2H-benzo[c]pyrrole de formule générale:

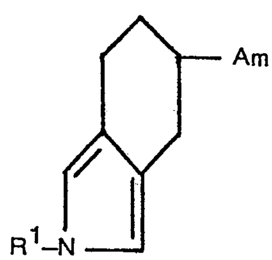

I

dans laquelle

$R^1$ représente H ou $R^2$;

Am représente $N(R^2)_2$; et chaque radical $R^2$ représente indépendamment un groupe allyle, un groupe méthyle, un groupe éthyle ou un groupe n-propyle; ainsi que de ses sels d'addition d'acide pharmaceutiquement acceptables, caractérisé en ce qu'il consiste à faire réagir un composé de 5-amino-4,5,6,7-tétrahydro-2H-benzo[c]pyrrole de formule:

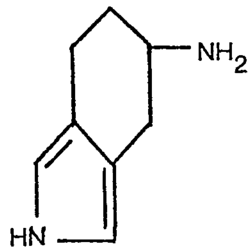

V

avec un aldéhyde approprié, effectuer une réduction avec un hydrure d'un métal ou avec un halolgénure d'allyle ou d'alkyle approprié et ensuite avec un halogénure d'acyle approprié, puis effectuer une réduction avec un hydrure d'un métal pour obtenir les composés de formule I dans laquelle $R^1$ représente H et Am représente $N(R^2)_2$; puis effectuer une réaction avec un halogénure d'alkyle et une base pour obtenir les composés de formule I dans laquelle $R^1$ représente $R^2$ et Am représente $N(R^2)_2$.

7. Composition pharmaceutique, caractérisée en ce qu'elle comprend un support et, comme ingrédient actif, un composé de formule I telle que définie dans l'une quelconque des revendications 1 à 5.

8. Composé répondant à la formule générale:

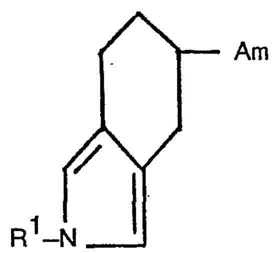

IA

dans laquelle

$R^1$ représente H ou

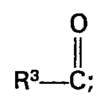

Am représente $NH_2$ ou

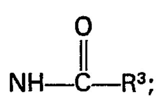

$R^3$ représente un groupe méthyle, un groupe éthyle ou un groupe n-propyle; avec cette exception que $R^1$ ne représente pas

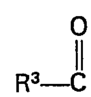

lorsque Am représente $NH_2$, ainsi que ses sels d'addition d'acide lorsque Am représente $NH_2$.

9. Composé répondant à la formule I telle que définie dans la revendication 1, ce composé étant préparé par le procédé suivant la revendication 6.

10. Composé de formule I destiné à être utilisé pour le traitement du syndrome de Parkinson et comme inhibiteur de prolactine.

**Patentansprüche**

1. Tetrahydro-2H-benzo[c]pyrrole der allgemeinen Formel I

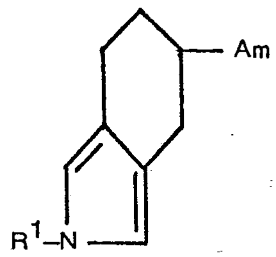

I

worin $R^1$ für H oder $R^2$ steht und

Am für $N(R^2)_2$ steht, wobei jeder der Substituenten $R^2$ unabhängig Allyl, Methyl, Ethyl oder n-Propyl bedeutet, und die pharmazeutisch unbedenklichen Säureadditionssalze hiervon.

2. Verbindung nach Anspruch 1, dadurch gekennzeichnet, daß jeder der Substituenten $R^2$ eine identische Gruppe bedeutet.

3. dl-5-Diethylamino-4,5,6,7-tetrahydro-2H-benzo[c]pyrrol.

4. dl-5-Di-n-propylamino-4,5,6,7-tetrahydro-2H-benzo[c]pyrrol.

5. dl-2-Methyl-5-di-n-propylamino-4,5,6,7-tetrahydro-2H-benzo[c]pyrrol.

6. Verfahren zur Herstellung von Tetrahydro-2H-benzo[c]pyrrolen der allgemeinen Formel I

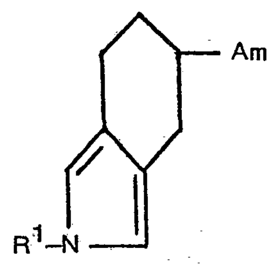

I

worin $R^1$ für H oder $R^2$ steht und

Am für $N(R^2)_2$ steht, wobei jeder der Substituenten $R^2$ unabhängig Allyl, Methyl, Ethyl oder n-Propyl bedeutet, und den pharmazeutisch unbedenklichen Säureadditionssalzen hiervon, dadurch gekennzeichnet, daß man ein 5-Amino-4,5,6,7-tetrahydro-2H-benzo[c]pyrrol der allgemeinen Formel V

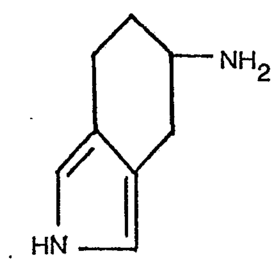

V

durch Umsetzen mit einem geeigneten Aldehyd und anschließende Reduktion mit einem Metallhydrid oder durch Umsetzen mit einem Allylhalogenid oder geeigneten Alkylhalogenid anschließende Umsetzung mit einem geeigneten Acylhalogenid und nachfolgende Reduktion mit einem Metallhydrid in Verbindungen der allgemeinen Formel I überführt, worin $R^1$ für H steht und Am für $N(R^2)_2$ steht, und die erhaltenen Verbindungen durch Umsetzung mit einem Alkylhalogenid und einer Base in Verbindungen der allgemeinen Formel I überführt, worin $R^1$ für $R^2$ steht und Am für $N(R^2)_2$ steht.

7. Pharmazeutische Zusammensetzung aus einem Träger und einem Wirkstoff, dadurch gekennzeichnet, daß sie als Wirkstoff eine Verbindung der allgemeinen Formel I gemäß einem der Ansprüche 1 bis 5 enthält.

8. Verbindung der allgemeinen Formel IA

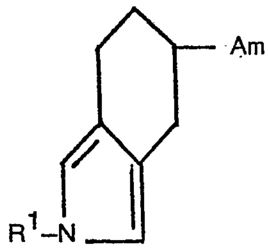

IA

worin $R^1$ für H oder

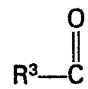

steht und

Am für $NH_2$ oder

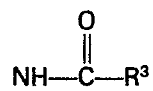

steht, wobei $R^3$ Methyl, Ethyl oder n-Propyl bedeutet, mit der Maßgabe, daß $R^1$ nicht

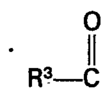

ist, falls Am für $NH_2$ steht, und die Säureadditionssalze hiervon, falls Am für $NH_2$ steht.

9. Verbindung der allgemeinen Formel I gemäß Anspruch 1, dadurch gekennzeichnet, daß sie nach dem Verfahren von Anspruch 6 hergestellt ist.

10. Verwendung einer Verbindung der allgemeinen Formel I zur Behandlung von Parkinsonscher Krankheit oder als Prolaktinhemmer.